# EUROPEAN PATENT SPECIFICATION

(11) **EP 2 827 813 B1**
(45) Date of publication and mention of the grant of the patent: **19.05.2021**
(21) Application number: 13721064.7
(22) Date of filing: 12.03.2013
(51) Int. Cl.: A61F 7/02, A61F 7/10

(54) **COLD THERAPY DEVICE**
KÄLTETHERAPIEVORRICHTUNG
DISPOSITIF DE THÉRAPIE PAR LE FROID

(30) Priority: 23.03.2012 US 201261614766 P
(43) Date of publication of application: 28.01.2015
(73) Proprietor: Angelini Pharma Inc., Rockville, MD 20850 (US)
(72) Inventor: EBEL, James Patrick, Lebanon, Ohio 45036 (US); FRANCO, Marle, Doral, Florida 33718 (US); LOOMIS, Erik Douglas, Loveland, Ohio 45140 (US)
(74) Representative: Colombo, Stefano Paolo
(86) International application number: PCT/IB2013/051953
(87) International publication number: WO 2013/140301

(56) References cited:
- EP-A1- 0 835 083
- WO-A1-2009/122336
- NL-C- 2 004 157
- US-A- 4 344 303
- US-A- 4 854 319
- US-A- 5 336 249
- US-A- 5 503 908
- US-A1- 2003 109 910
- US-A1- 2003 124 277

## Description

### FIELD OF THE INVENTION

The present invention is directed to a device for absorbing heat from a body. More particularly, the invention pertains to an improved device which utilizes a gel material comprising liquids and solids to absorb, over an extended period of time, heat from a body.

### BACKGROUND

Cold therapy has been a recommended treatment for pain for decades. Commonly, this therapy was provided via an ice bag which was held against the injured or painful area. More recently, improved devices have been developed for use in providing cold therapy.

Reusable cold packs generally contain a viscous solution, suspension, or gel, mostly aqueous or glycol-based, that is cooled or frozen in a refrigerator or freezer. Those that do not freeze at typical household freezer temperatures, or have less frozen material than pure water, have a reduced capacity to absorb heat compared to those that do freeze or have a greater proportion of water, and thus have a shorter duration. This is because frozen cold packs can absorb the heat of fusion or melting of ice which is much greater than the latent heat required to raise the temperature of an unfrozen liquid. A more desirable device has a greater proportion of frozen material or is entirely frozen, thus increasing its capacity to absorb heat.

In addition, frozen solutions, suspensions, or gels maintain a relatively constant temperature during use at the freezing point of the solution, suspension, or gel. The constant temperature region of the profile over time is determined by the melting point of the solution, suspension, or gel, and varies with composition. Many solutions, suspensions, or gels contain additives that depress the freezing point substantially below the freezing point of water. This can lead to skin temperatures below freezing when applied to the body. A more desirable temperature profile has a safe and comfortable yet effective temperature that is controlled by the melting point of the solution, suspension, or gel, and is close to the freezing point of water.

To address the safe and comfortable skin temperature that is not too cold, most current products recommend that a towel or some other piece of fabric be placed between the cold product and the skin. The limitation of this approach is that the resulting skin temperature is highly variable and determined by the material and thickness of the towel or fabric rather than the device. Other products provide a cover or wrap for the cold pack when applied to the body. These often provide an arbitrary level of insulation, and are not designed to give a specific skin temperature. They are also uniform in material and construction on both the clothing-side and body-side of the product which does not differentially control heat flow from the surrounding air and the body. A more desirable design minimizes heat absorption from the surroundings, and controls heat absorption from the body through the selection of the insulation factors of the clothing-side and body-side materials.

One conventional cold pack is marketed under the trademark "THERAPAC®" and comprises a 30 cm by 30 cm (12 inch-by-12 Inch) two-ply, vinyl pack filled with a white, odorless, insoluble gelatin. Another conventional cold pack is marketed under the trademark "COLPAC®" and comprises a 30 cm by 30 cm (12 inch-by-12 inch) single-ply, plastic pack filled with clay. Such conventional cold packs are widely disseminated and absorb heat. One principal disadvantage of such cold packs is that they have a relatively limited ability to absorb heat compared to frozen water. For example, when the THERAPAC® and COLPAC® cold packs noted above are removed from a freezer, they warm up quickly. Compared to devices that contain substantially frozen material, these are at a temperature above the therapeutic range at much shorter times.

Another conventional cold pack is marketed under the trademark "CRYOMAX®" and comprises a rectangular single-ply plastic pack consisting of two types of chambers. One chamber is filled with a propylene glycol and water mixture, and the other chamber contains substantially pure water. This particular product stays cold longer than the THERAPAC® and COLPAC® devices since one of its components is frozen water, but it still includes a significant amount of unfrozen material. This renders the product bulky, heavy, and cumbersome to use with limited ability to fit well to many body parts.

Currently marketed cold packs are not designed to deliver a specific device and skin temperature profile over time, and are often difficult or uncomfortable to apply to the body. Control of the temperature profile addresses a need to give a safe and comfortable skin temperature that is not too cold, and provides an effective, therapeutic temperature for the desired duration. Accordingly, it is highly desirable to provide an improved cold pack that during application to the body, maintains a defined therapeutic temperature for an extended period of time, and is easy to apply to the body. This is highly desirable as it reduces user confusion about the proper temperature, reduces or eliminates the uncomfortable feeling of applying cold to the body, and makes it easier and more convenient to apply the device NL2004157, US 2003/124277, US 5503908, US5336249 and EP0835083 disclose prior art documents relevant for the present invention.

### SUMMARY OF THE INVENTION

The present invention is defined by the appended claim 1. Preferred embodiments are disclosed in the dependent claims. The invention comprises a multi-use cold pack device comprising an attachment wrap comprising a Z-configuration and having a highly adjustable fastening zone, and further comprising a corrugated material; a body-side insulation layer; a clothing-side insulation layer; and a multi-cell gel composition, wherein said gel composition does not include an antifreeze component.

In still another embodiment, the invention comprises a multi-use cold pack device comprising an attachment wrap comprising a corrugated material and having a highly adjustable fastening zone; a body-side insulation layer; a clothing-side insulation layer; and multi-cell gel composition, wherein said gel composition does not include an antifreeze component.

In a further embodiment, the invention comprises a multi-use cold pack device as herein described, wherein the R-value of said body-side insulation layer is less than the R-value of said clothing-side insulation layer.

In another embodiment, the invention comprises a multi-use cold pack device as herein described, wherein the body-side insulation layer has an R-value of 0.001 m²-K/W to 0.1 m²-K/W and the clothing-side insulation layer has an R-value that is greater than 0.1 m²-K/W.

In still another embodiment, the invention comprises a multi-use cold pack device as herein described, wherein the body-side insulation layer has an R-value of 0.001 m²-K/W to 0.01 m²-K/W and the clothing-side insulation layer has an R-value that is greater than 0.01 m²-K/W.

In a further embodiment, the invention comprises a multi-use cold pack device as herein described, wherein the gel composition freezes into a solid form at a temperature between -5°C to 5°C. The invention comprises a multi-use cold pack device as herein described, wherein the corrugated material uniformly stretches to no more than about 5% to about 15% necking, or anywhere within the about 5% to about 15% necking range.

In yet another embodiment, the invention comprises a multi-use cold pack device as herein described, wherein the corrugated material uniformly stretches to no more than 10% necking.

In still another embodiment, the invention comprises a multi-use cold pack device as herein described, wherein the corrugated material uniformly stretches to no more than 5% necking.

In a further embodiment, the invention comprises a multi-use cold pack device as herein described, wherein the gel composition, when placed in contact with a user's skin, reduces said skin temperature by at least 10°C.

In another embodiment, the invention comprises a multi-use cold pack device as herein described, wherein the gel composition, when placed in contact with a user's skin, maintains a reduced temperature of said user's skin for about 15 minutes to about 60 minutes.

In another embodiment, the invention comprises a multi-use cold pack device as herein described, wherein the gel composition, when placed in contact with a user's skin, maintains a reduced temperature of said user's skin for at least about 15 minutes.

In yet another embodiment, the invention comprises a multi-use cold pack device as herein described, wherein the gel composition further comprises a thermochromic dye.

In still another embodiment, the invention comprises a multi-use cold pack device as herein described, wherein each of the individual cells of said gel composition have a thickness of about 10 mm, or about 2 mm to 20 mm.

### BRIEF DESCRIPTION OF THE DRAWINGS

**Figure 1 (FIG 1****)** is a perspective view of an embodiment.
**Figure 2 (FIG 2****)** is a perspective view of an embodiment with a segmented wrap configuration.
**Figure 3 (FIG 3****)** is a perspective view of an alternative embodiment of the invention which shows the Z configuration.
**Figure 4 (FIG 4****)** depicts an experimental setup used for the *in vitro* testing for the gel composition samples.
**Figure 5 (FIG 5****)** depicts an enlarged cross-sectional view of a gel pack sample with surrounding insulation layers. This sample is used for the *in vitro* experimental setup.
**Figure 6 (FIG 6****)** is a graph showing the *in vitro* skin temperature analysis for gel compositions with varying percentages of propylene glycol.
**Figure 7 (FIG 7****)** is a graph showing the *in vitro* product temperature analysis for sampled gel compositions with varying percentages of propylene glycol.
**Figure 8 (FIG 8****)** is depiction of a sample thermal image taken during *in vivo* human testing showing the skin temperature during cold treatment.
**Figure 9 (FIG 9****)** is a graph showing the *in vivo* average device temperature analysis of an embodiment of the gel composition.
**Figure 10 (FIG 10****)** is a graph showing the *in vivo* average IR skin temperature analysis of an embodiment of the gel composition.
**Figure11 (FIG 11****)** is a graph showing the *in vivo* average IR skin temperatures of representative embodiments D1, D2 and D3 of the invention in comparison with an ACE® wrap, a CRYOMAX® wrap, and an ice bag.

### DETAILED DESCRIPTION OF THE INVENTION

### DEFINITIONS

"Antifreeze", "antifreeze additive", or "antifreeze component" are terms which refer to a material added to a composition that lowers the freezing point of the composition, or interferes with solidification during freezing. Examples of such materials that resist freezing are propylene glycol, ethylene glycol, polyethylene glycol, similar alcohol-based components, glycerin, and the like known in the art.

"Attachment wrap" refers to all components of a cold therapy device, other than the gel and gel packs, used to attach the device to a body or inanimate object. The attachment wrap may be combined with the multi-cell gel pack located between the two segments of stretch material to yield the present invention. The attachment wrap is optionally referred to as a "belt-like wrap" or "wrap" in one or more representative embodiments of the invention.

"Body-side" refers to the surface of a gel pack, wrap, or device that is intended to face and contact the skin or body of the user during wear.

"Clothing-side" refers to the surface of a gel pack, wrap, or device that is intended to face away from the skin or body of the user during wear. The term "clothing-side insulation layer" refers to the orientation as being on the side not in contact with the user's skin.

"Cold pack device" refers to the unit comprising a cold pack or cold packs, and an attachment wrap for applying the device to the target area, such as a user's body. "Cold pack device" may also be referred to as "cold therapy device" or "cold therapeutic device" in one or more embodiments of the invention.

"Fit range" describes the circumference of the body part that the attachment wrap will fit. The term fit range includes circumference measurements for humans and non-human animals.

"Gel" is used broadly to describe the contents of a gel pack and is the cooled, partially frozen, or frozen material that absorbs heat in a cold therapy device according to the present invention. As used herein, gel compositions include, but are not limited to, liquids, thickened or gelled liquids, suspensions, dispersions, semisolids, solid dispersions, and solids.

"Gel pack" is used broadly to describe the collection of cells, compartments, containment structures, pouches, or pouch-like structures that contain the gel in a cold therapy device; the gel pack provides the cooling effect to the body during wear of a cold therapy device. "Gel pack" may also be referred to as "cold pack" in one or more embodiments of the invention.

"Multi-cell" is used to indicate more than one compartment, containment structure, pouch, or pouch-like structure that contains the gel in a cold therapy device.

" Fastening zone" refers to any part of the clothing-side of the device that will engage the hook component of the hook and loop fastening system.

"Highly adjustable fastening zone" refers to a fastening zone where a majority or the entirety of the clothing-side of the device will engage the hook component of the hook and loop fastening system.

"Necking" (or neck down) is the effect that results in the difference in width of the stretch portion of the attachment wrap as it transitions from a relaxed state to an active state.

"Relaxed state" refers to the condition of the attachment wrap while not being worn (i.e., in un-stretched state)

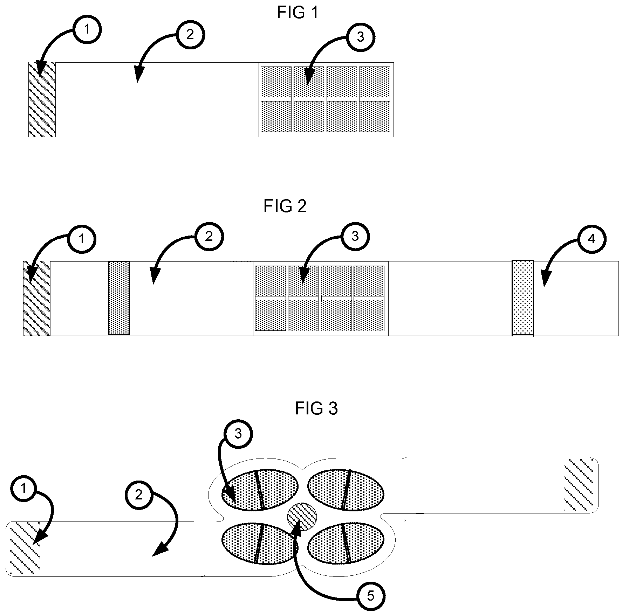

"Active state" refers to the condition of the attachment wrap while being worn (i.e., stretched).
"in." refers to inches.
"mm" refers to millimeters.
"m²-K/W" refers to meters squared times degrees Kelvin divided by watts.
"time-Temperature" refers to temperature measurements made over a period of time, and temperature as a function of time.
"Insulation" refers to material that impedes or reduces the flow of heat to and from an object, including the body of a user or a cold pack.
"R-value" is a measure of resistance to heat flow through a given thickness of material. It is used to assign an insulation value to a given material. Under uniform conditions, it is the ratio of the temperature difference across an insulator and the heat flux through the material. It is expressed as the thickness of the material divided by the thermal conductivity of the material.
"Thermochromic Dye" refers to temperature sensitive dye that changes color based on the temperature.
"Multi-use" refers to the intent that the device be used more than once.
"IR" refers to infra-red.
"FLIR" refers to forward looking infra-red. The "Z-configuration" according to the present invention refers to an attachment wrap having a non-linear configuration extending from opposing sides of the gel pack. A graphic example of one embodiment of a Z-configuration is presented in Figure 3 of the present application. The term "Z-configuration" may also be referred to as "Z-strap configuration" or an "offset configuration" in one or more embodiments of the invention.

All percentages, parts and ratios are by weight, unless otherwise specified. All such weights as they pertain to listed ingredients and components are based on the specific ingredient level and, therefore, do not include carriers or by-products that may be included in commercially available materials, unless otherwise specified.

The present invention is a cold therapy device that is easily and comfortably worn on the body, and provides a relatively constant, safe, and therapeutic tissue temperature for an extended period of time. This performance is achieved through an ergonomic and flexible wrap design that keeps the device in place and in good contact with the body, a gel pack composition that, when frozen, maximizes duration at a constant temperature, a first insulation layer on the clothing-side or outside of the wrap that minimizes heat influx from the surroundings, and a second insulation layer on the body-side or body contact surface that contributes to the safe and effective tissue temperature. Because heat transfer to a cold wrap in contact with the body is dynamic, all of these elements must work in concert to provide a prescribed temperature and thermal performance.

This cold therapy device is applied to the user's body surface. The body parts to which the device is applicable include, but are not limited to, the lower back, the abdomen, the neck, shoulders, knees, elbows, and various other joints, and the like, as desired. One or more exemplary embodiments of the device may also be used on non-human animals, for example, in veterinary applications where cold therapy is desired for treating various domesticated or wild animals.

The invention comprises a flexible, segmented, wearable cold wrap that has insulation layers on both sides of the gel pack. The clothing-side insulation reduces absorption of heat from the surroundings, thus maximizing the amount of heat that can be absorbed from the body and increasing the duration of the safe and effective temperature performance. The body-side insulation layer regulates the thermal conduction between the body and the cold pack such that a safe, effective, and comfortable tissue temperature is experienced by the user. The insulation value of the body-side layer is specifically selected to give the proper tissue temperature given the frozen gels' melting point.

The flexible, insulated, segmented cold pack can be configured to wrap around any body part and stay in place. It is common for other gel pack products to require a secondary component as an attachment device. For many gel packs, an elastic bandage is used as a secondary attachment device. Some other gel packs are accompanied by a recommended holder or sleeve that requires assembly of components. These attachment devices are not specifically designed to provide the required safe and effective tissue temperatures. When an attachment device is not provided, it is recommended that the user wrap the cold pack in a cloth or towel. Since the tissue temperature depends on the insulation value of the attachment device, cloth or towel, the resulting tissue temperature is variable and not optimal.

The cold pack device according to the invention is lightweight, and flexible, while simultaneously allowing stretch and recovery in areas of the user such as shoulders, elbows, knees, neck, and the like. Even after frequent utilization, the cold pack device has excellent extensibility and still maintains its shape.

This invention includes a cold wrap that has distinct body-side and clothing-side insulation on the opposing surfaces of the gel pack. A major advantage of one embodiment of the invention is that it excludes the requirement of a secondary attachment device. For two-part devices, the attachment device is stored separately from the gel pack and the two parts must be assembled prior to use. The invention method-of-attachment is useful since the devices can be attached to the body while maintaining mobility of the user enabling the user to move about during wear. One embodiment of the present invention is a single unit that simplifies the storage, preparation, and application of the wrap by the user compared to common methods of attachment.

In one or more embodiments, the clothing-side appearance of the cold pack device can be a printed logo, a sports team emblem, a graphic or other design, and any color or combination(s) thereof. The clothing-side appearance may also be unprinted material and is easily customizable to a supplier's preference. The body-side appearance may also be printed, but typically is not seen when applied to a user and thus may be unprinted.

The invention comprises a Z-configuration with a gel pack preferably comprising 4 subdivided ovals having 8 individual cells. In another embodiment, the invention comprises a Z-configuration with a gel pack comprising 4 circular discs.

The invention comprises a continuous belt-like wrap with a gel pack comprising 2 sets of 4 square gel cells (for a total of 8 cells) comprising the gel pack. This representative embodiment may be used, for example, in fastening the cold pack device around a user's waist, leg, or other large body area.

**FIG 1** provides an embodiment wherein the cold therapy device has continuous areas of stretch 2A material on both sides of the cold pack. The gel pack 3A is located in the center of the device between the two segments of stretch 2A material. The stretch 2A areas allow the wrap to be applied with the proper amount of force to keep it in place during wear, and remaining comfortable to the user. The wrap is fastened using a hook 1A and loop system. On one end of the wrap the hook 1A is attached, and the loop or fastening zone can be found across the entire clothing-side of the wrap. This allows the hook 1A to attach at any point on the wrap (also referred to as a highly adjustable fastening zone), resulting in a wide fit range.
**FIG 2** provides an embodiment wherein the cold therapy device has several discrete areas of stretch 2A and non-stretch 4A materials on both sides of the wrap. The gel pack 3A is in the center of the device. The non-stretch 4A material may also be referred to as an extender, or a non-stretch extender. The stretch 2A areas allow the wrap to be applied with the proper amount of force to keep it in place during wear, and remaining comfortable to the user. The wrap is fastened using a hook 1A and loop system. On one end of this wrap the hook 1A is attached, and the loop or fastening zone can be found across the entire clothing-side of the wrap. This allows the hook 1A to attach at any point on the wrap, resulting in a wide fit range.
**FIG 3** provides an embodiment of the invention wherein the wrap has straps on opposite ends of the gel pack 3A area that are offset from the center-line along the length of the wrap. The hook 1A and loop fastening system works similarly to the embodiments in **FIG 1** and **FIG 2****,** and the loop or fastening zone can be found across the entire clothing-side of the wrap. However, in this embodiment, the hook 1A is attached to the ends of both straps. This allows the straps to be pulled in opposite directions and independently attached to the loop or fastening zone resulting in a wide fit range. The two-strap design also allows for the straps to be fastened above and below a joint, for example, preserving the user's mobility and keeping the wrap in place during wear. The cut-out 5A allows for the wrap to be secured to/around joints without limiting the range of mobility.

The Z configuration allows the user to apply the device to the desired area of the body using only one hand to fasten the first strap, then adjust the device and using only one hand to fasten the other strap for the final fit. Further, the Z configuration allows a user to pull the offsetting straps in opposite directions while applying the device, which action keeps the device in place as desired and avoids any rotation of the device while being applied to the desired area.

In a further embodiment, the highly adjustable fastening zone enables a fit range of approximately 51 cm to 203 cm (20 to 80 inches). In another embodiment ("Joint"), the highly adjustable fastening zone enables a fit range of approximately 51 to 127 cm (20 to 50 inches). In yet another embodiment ("Muscle"), the highly adjustable fastening zone enables a fit range of approximately 51 to 193 cm (20 to 76 inches).

### Gel Pack Design/Composition

The gel pack comprises a freezable composition contained in a pouch or pouch-like containment structure(s). The freezable composition may be, but is not limited to, a liquid, thickened or gelled liquid, suspension, or a solid, or solid suspension, or mixtures of these. These freezable compositions are generically referred to as "gel" or "gels" in this disclosure.

The gel pack containment structure may be composed of a strong, durable, rigid, semi-rigid, flexible, malleable, conforming, and/or plastic-like material. The gel pack consists of multiple containment compartments. Furthermore, the gel pack and compartments of the gel pack may be designed with any shape, such as oval, circle, square, rectangular, multi-cell, and other shapes. Compartments and shapes are generated by sealing the perimeter of the compartment or compartments. This segmented, multi-compartmental design with sealed joints allows the gel pack to bend and conform to the body treatment area. Further, the segmented, multi-compartment design avoids the formation of a solid ice block which reduces contact and comfort (a known problem in the art for solid ice blocks). The multi-cell design also helps maximize contact between the skin surface area with the individual cells of the gel pack.

The gel pack may or may not be permanently enclosed into the wrap. For gel packs that are not permanently enclosed, the wrap may include an access apparatus such as a zipper, hook, buttons, straps, and/or snaps, or the like, to allow for placement and removal of the gel pack. In this manner, the gel pack may be replaced if necessary.

The gel composition may include any material that provides a cooling effect to the body when frozen. For example, the gel may include, but is not limited to, a liquid, thickened or gelled liquid, suspension, or a solid, or solid suspension, or mixtures of these. The gel compositions are typically water-based. The gel composition may also include natural, synthetic, food grade and/or thermochromic dyes to indicate the temperature or frozen state of the gel pack. Many gel packs have gel compositions that contain antifreeze additives that significantly reduce the freezing point, or prevent complete freezing or solidification of the gel. This allows the gel pack to remain malleable when frozen. Examples of these antifreeze additives are propylene glycol and glycerine. The gel compositions disclosed do not contain antifreeze additives.

A limitation of gel compositions that do not entirely freeze is that the gel has less heat absorption capacity than pure frozen water. A preferred gel composition is one that minimizes the amount of unfrozen gel to maximize heat absorption capacity and thus maximize the duration of the gel pack.

An additional limitation of gel packs having gel compositions that contain antifreeze additives is that the freezing temperature of the gel may be significantly lowered compared to the freezing temperature of water. Lower gel freezing temperatures increase the likelihood of producing tissue temperatures below a safe value. A preferred water-based gel composition is one that has a freezing point that is at or near the freezing temperature of pure water.

### EXAMPLES

The following examples further describe and demonstrate embodiments within the scope of the present invention. The examples are given solely for the purpose of illustration and are not to be construed as limitations of the present invention, as many variations thereof are possible without departing from the scope of the appended claims. All exemplified concentrations are weight-weight percents, unless otherwise specified.

### In vitro Laboratory Testing Samples

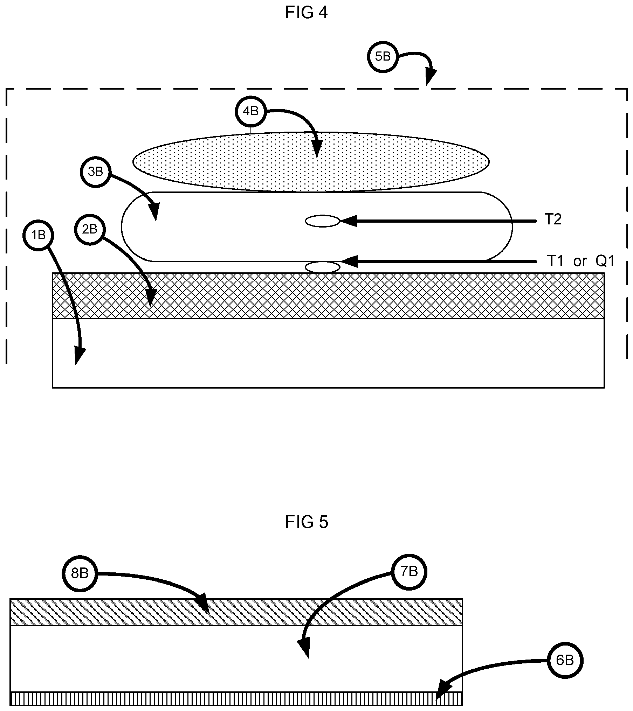

FIG 5 depicts a gel pack sample used in the *in vitro* laboratory testing setup. Two pieces of Golden Eagle HB 3.5 LLPDE were sealed together using an impulse sealer in order to make the containment structure for several gel packs. On the clothing-side of the pack is an insulation layer 8B (i.e., clothing-side insulation layer), and on the body-side of the pack is insulation layer 6B (i.e., body-side insulation layer). The frozen gel pack was placed over an array of thermocouples that lay on top of a heated Lexan plate surface. Gel packs were made containing each of the following gel compositions:

| Sample Label | Gel Composition (percents are by weight) |
|---|---|
| F | 0% propylene glycol in water mixture |
| G | 10% propylene glycol in water mixture |
| H | 20% propylene glycol in water mixture |
| I | 30% propylene glycol in water mixture |
| J | 40% propylene glycol in water mixture |

### In vitro Laboratory Testing

The testing setup provided in **FIG 4** was used to measure the temperature versus time profile between the gel pack body-side surface and Lexan plate surface for various gel compositions 7B. The sample 3B was placed on the test surface 2B (also referred to as the Lexan plate surface) which is in contact with circulating water bath 1B which provides a constant temperature heat sink to deliver heat to the cold pack. A weight 4B was placed on top of the sample gel pack to simulate wrap compression and give good contact among all of the surfaces. The full testing setup was enclosed 5B in order to control the environmental conditions of the system.

Two temperature measurements were taken: T1, the temperature between the bottom or body-side surface of the experimental sample and the Lexan plate surface 2B (simulating skin temperature, and referred to as the contact temperature), and T2, the internal temperature or gel temperature of the sample (referred to as the product temperature). Also, a heat flux measurement was taken: Q1, which was the heat transfer rate between the sample 3B and the Lexan plate surface 2B. The heat flux measurement, Q1, gives the rate that the gel pack is absorbing heat.

The sample depicted in **FIG 5** shows the structure of the gel pack composition 7B used to collect the temperature versus time data for each sample that was prepared. The clothing-side of the gel pack has clothing-side insulation 8B that reduces heat absorption from the surrounding air. The body-side insulation 6B is sufficient to modify the contact temperature to a value greater than the gel pack temperature.

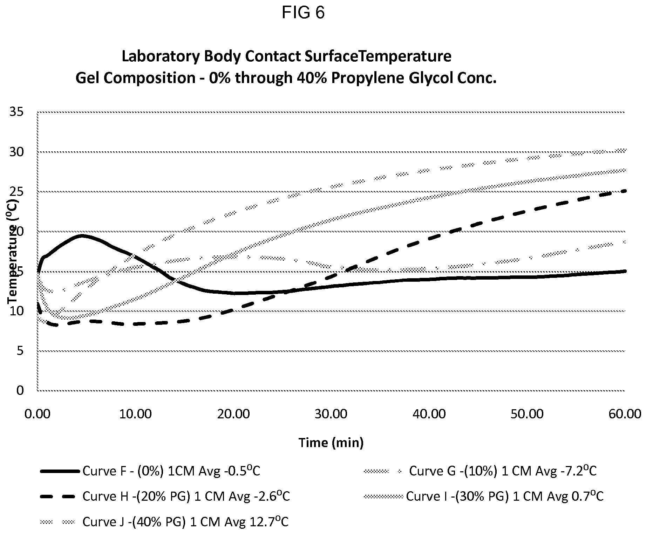

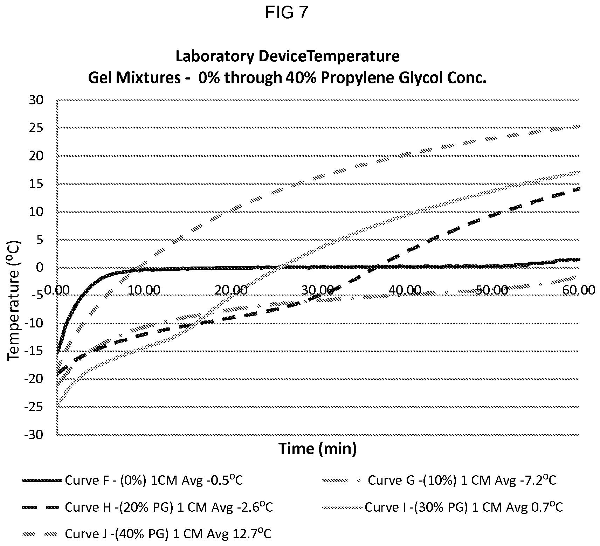

**FIG 6** and **FIG 7** depict the results for sample contact temperature T1 and the product temperature T2, respectively, for all the samples that were prepared: F, G, H, I, J and K. The data show the effect of including an antifreeze additive, e.g., propylene glycol, in the gel composition. Increasing amounts of antifreeze additive reduce the contact temperature and product temperature below the freezing point of water for significant periods of time. During wear by a user, temperatures below freezing represent a potential safety risk to the tissue. In addition, the data show that increasing amounts of antifreeze additive reduce the length of time over which a constant contact and product temperature can be maintained.

### In vivo Human Testing Samples

The embodiments of the cold therapy device shown in **FIG 1** and **FIG 3** were used to generate data during *in vivo* testing on human subjects. The gel composition tested contained 0% propylene glycol to maximize the amount of frozen water and maintain a gel pack melting or freezing temperature near that of pure water of 0°C.

### In vivo Human Testing

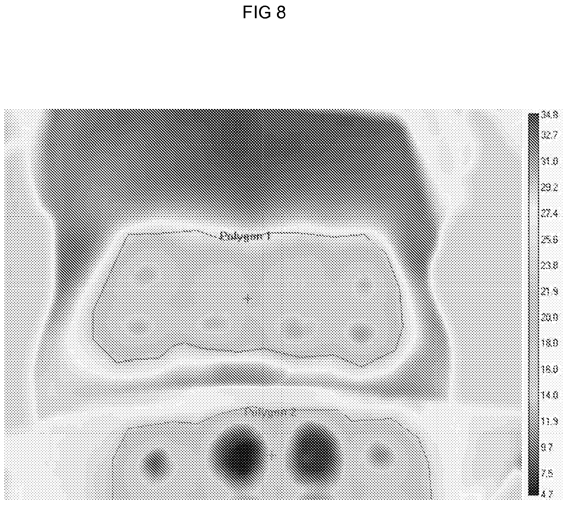

Temperature versus time data was collected from human subjects wearing the cold therapy devices. The sample wraps were placed on the subject's body along with thermocouples between the body-side of the cold pack and the subject's skin to capture the skin temperature, and on the clothing-side of the gel pack surface to capture the product temperature. Skin and product temperatures were recorded continuously for one hour of wear. Infrared (IR) camera measurements were taken at ten minute intervals to verify both skin and product temperatures. An example of an infrared image is shown in **FIG 8****.**

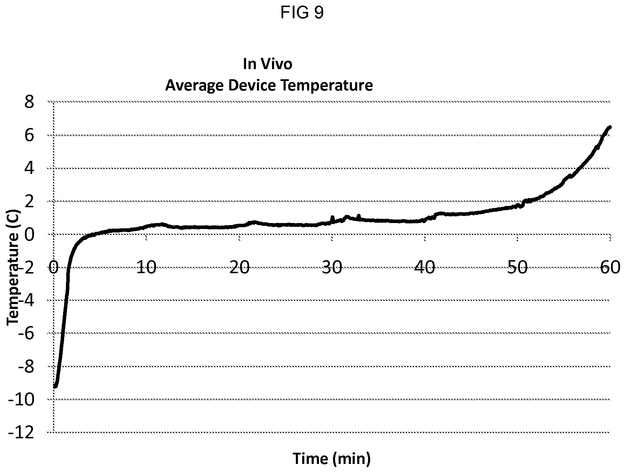

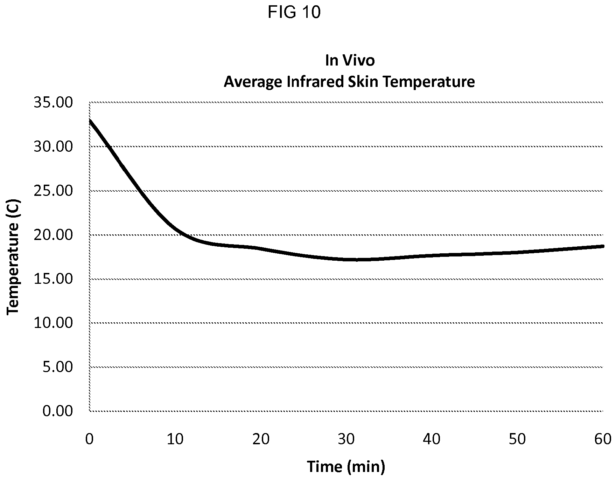

This testing setup was used to generate time-temperature profiles that are shown in **FIG 9** (IR for skin) and **FIG 10** (thermocouple for product). These time-temperature profiles demonstrate that the cold therapy device was able to maintain a relatively constant skin temperature of less than 20°C for nearly one hour, and maintain a relatively constant product temperature between 0°C and 5°C for nearly one hour. These temperatures are indicative of safe and effective performance.

A review of the literature on the effectiveness of cold therapy in relieving pain and swelling suggests preferred temperature targets for cold therapy devices. The first target is achieving a tissue temperature reduction of >10°C for a minimum of 20 minutes. The second target is achieving a device temperature of 0°C to 5°C during the treatment period. These temperatures are generally reflective of the performance of an ice bag, a commonly used cold therapy device in efficacy studies.

### Figure 6. Laboratory Device Body Contact Surface Temperature

Laboratory data using a simulated wear condition shows that for an example embodiment having a gel composition with no anti-freeze agent, the surface of the device that contacts the body remains at a relatively constant temperature during a 1-hour time period. In contrast, devices having a gel that contains an anti-freeze agent result in body contact surface temperatures that vary with time.

### Figure 7. Laboratory Device Temperature

Laboratory data using a simulated wear condition shows that for an example embodiment having a gel composition with no anti-freeze agent, the device temperature remains at a relatively constant temperature during a 1-hour time period. The temperature achieved by the example gel composition closely adheres to the preferred temperature range of 0°C to 5°C. In contrast, devices having a gel that contains an anti-freeze agent result in temperatures that vary with time, and deviate substantially from the preferred temperature range of 0°C to 5°C.

### Figure 9. In Vivo Device Temperature

When human subjects wear the device of an example embodiment, the device temperature closely mimics the laboratory data and largely remains within the preferred temperature range of 0°C to 5°C.

### Figure 10. In Vivo IR Skin Temperature

When human subjects wear the device of an example embodiment, the resulting skin temperature closely mimics the laboratory data and largely achieves the preferred skin temperature decrease of >10°C.

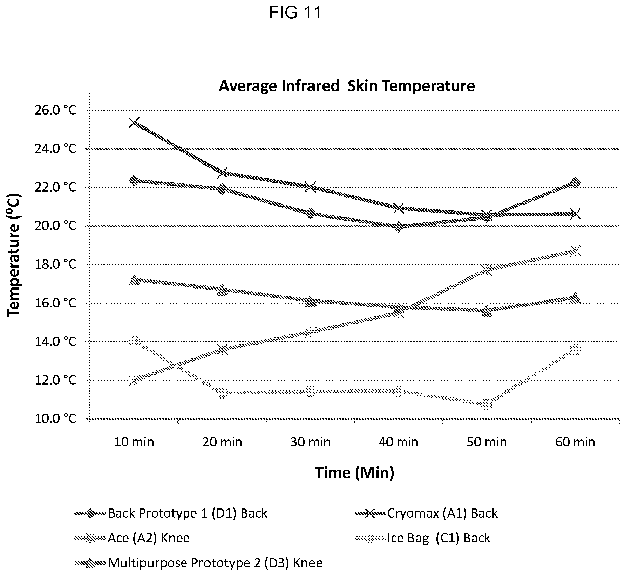

### Figure 11. In Vivo IR Skin Temperature

When human subjects wear the device of an example embodiment, the resulting skin temperature is relatively constant when compared to two marketed devices. The CryoMax® device exhibits a temperature profile that declines with time, and the ACE® device exhibits a temperature profile that increases with time. The constant temperature profile is preferred.

### OPTIONAL COMPONENTS

The compositions of the present invention can further comprise one or more optional components known or otherwise effective for use in such compositions, provided that the optional components are physically and chemically compatible with the compositional components described hereinabove, or do not otherwise unduly impair product stability, aesthetics, or performance.

In an embodiment, the center hole in the gel pack accommodates better custom fit for a user, such as by wrapping the attachment strap ends around a user's mid portion of the leg while leaving the kneecap exposed/uncovered by the gel pack.

In an embodiment, the user is not required to have the cold pack device at the lowest possible temperature point (e.g., the multi-cell gel pack in a frozen state) because the user can still apply the cold pack device to the user's desired area in order to withdraw heat and thus cool the target area of the user's body. For example, an associated advantage of using the cold pack device at less-than-coldest temperature helps to ensure compliance with more sensitive users, such as children, elderly patients, sensitive animals, and the like. Also, the insulating layer provides better compliance among animals (e.g., in veterinary medicine use) that similarly react by recoiling from application of the cold pack to the skin.

In another embodiment, one may use the cold pack device according to the invention by applying it to moderately burned areas, such as for providing relief from moderate sunburn.

In one embodiment, the wrap is constructed from a nonwoven, corrugated material that can stretch with minimal necking and recover in shape to substantially conform to the skin area of the user. Accordingly, the cold pack device conforms to irregular 3-dimensional body areas to maintain the interface of the gel pack cells with the desired area to be cooled by the cold pack. This unique feature occurs regardless of whether the individual cold pack gel cells are completely frozen or are in a liquid state.

The body-side insulation layer of the present device minimizes (or even eliminates) the initial shock of applying a cold/frozen pack to a user's skin. This body-side insulation layer of the present invention provides a unique solution to the known "shock sensation" problem that typically occurs when suddenly placing an extremely cold (or even frozen) cold pack directly against the user's bare skin. As discussed, simply placing a towel or other layer(s) between the ice pack and the user's skin is an ineffective approach that substantially reduces the desired cooling effect of the ice pack and is very inefficient at cooling the target area.

In contrast, the body-side insulation layer of the present invention does not create a shock sensation when placing the cold pack device against the user's skin. Further, once placed against the user's skin, the present device maintains a cooling of the user's skin and enables the user to maintain the contact of the present cold pack device for an extended period without causing discomfort to the user. The body-side insulation layer further prevents damaging the user's skin where an ice pack placed in direct contact with the skin for an extended period of time could ordinarily cause a cold burn and even produce frostbite of the area in extreme cases.

While particular embodiments of the present invention have been illustrated and described, it would be obvious to those skilled in the art that various other changes and modifications can be made without departing from the scope of the appended claims.

## Claims

1. A multi-use cold pack device comprising:
(i) an attachment wrap comprising a Z-configuration and having a highly adjustable fastening zone; and
(ii) a multi-cell gel pack (3) located between two segments of stretch material of the attachment wrap, including
- a body-side insulation layer (6B);
- a clothing-side insulation layer (8B) extending opposite to the body-side insulation layer; and
- a gel composition (7B) arranged between said body-side and clothing-side insulation layers, wherein said gel composition does not include an antifreeze component,
**characterized in that** the two segments of stretch material each comprises a corrugated material that uniformly stretches to no more than 15% necking.

2. A multi-use cold pack device according to claim1, wherein the thickness of said body-side insulation layer **(6B)** is thinner than the thickness of said clothing-side insulation layer **(8B).**

3. A multi-use cold pack device according to claim 1 or 2, wherein said body-side insulation layer **(6B)** has an R-value of 0.001 m²-K/W to 0.01 m²-K/W.

4. A multi-use cold pack device according to claim 1, wherein said clothing-side insulation layer **(8B)** has an R-value that is greater than 0.01 m²-K/W.

5. A multi-use cold pack device according to claim 1, wherein said body-side insulation layer **(6B)** has an R-value of 0.001 m²-K/W to 0.01 m²-K/W and said clothing-side insulation layer **(8B)** has an R-value that is greater than 0.01 m²-K/W.

6. A multi-use cold pack device according to claim 1, wherein said gel composition **(7B)** freezes into a solid form at a temperature between -5°C to 5°C.

7. A multi-use cold pack device according to claim 1, wherein said corrugated material uniformly stretches to no more than 10% necking.

8. A multi-use cold pack device according to claim 1, wherein said corrugated material uniformly stretches to no more than 5% necking.

9. A multi-use cold pack device according to claim 1, wherein said gel pack (3) when placed in contact with a user's skin reduces said skin temperature by at least 10°C.

10. A multi-use cold pack device according to claim 1, wherein said gel pack (3) when placed in contact with a user's skin maintains a reduced temperature of said user's skin for about 15 minutes to about 60 minutes.

11. A multi-use cold pack device according to claim 1, wherein said gel pack (3) when placed in contact with a user's skin maintains a reduced temperature of said user's skin for at least about 15 minutes.

12. A multi-use cold pack device according to claim 1, wherein said gel composition further comprises a thermochromic dye.

13. A multi-use cold pack device according to claim1, wherein each of the individual cells of said gel pack (3) have a thickness of about 2 mm to 20 mm.

## Patentansprüche

1. Mehrfach verwendbare Kältepackungsvorrichtung, umfassend:
(i) ein Befestigungsband mit Z-Konfiguration und einer hochanpassbaren Befestigungszone;
(ii) eine zwischen zwei Segmenten Stretchmaterial des Befestigungsbandes angeordnete mehrzellige Gel-Packung (3), einschließlich
- einer körperseitigen Isolationsschicht (6B);
- einer kleidungsseitigen Isolationsschicht (8B), die sich gegenüber der körperseitigen Isolationsschicht erstreckt; und
- einer zwischen der körperseitigen und der kleidungsseitigen Isolationsschicht angeordneten Gelzusammensetzung (7B), wobei die Gelzusammensetzung keine Frostschutzmittelkomponente enthält,
**dadurch gekennzeichnet, dass** die zwei Segmente Stretchmaterial ein gewelltes Material umfassen, das sich gleichmäßig auf nicht mehr als 15 % Einschnürung dehnt.

2. Mehrfach verwendbare Kältepackungsvorrichtung nach Anspruch 1, wobei die Dicke der körperseitigen Isolationsschicht (6B) dünner ist als die Dicke der bekleidungsseitigen Isolationsschicht (8B).

3. Mehrfach verwendbare Kältepackungsvorrichtung nach Anspruch 1 oder 2, wobei die körperseitige Isolationsschicht (6B) einen R-Wert von 0,001 m²-K/W bis 0,01 m²-K/W aufweist.

4. Mehrfach verwendbare Kältepackungsvorrichtung nach Anspruch 1, wobei die kleidungsseitige Isolationsschicht (8B) einen R-Wert aufweist, der größer als 0,01 m²-K/W ist.

5. Mehrfach verwendbare Kältepackungsvorrichtung nach Anspruch 1, wobei die körperseitige Isolationsschicht (6B) einen R-Wert von 0,001 m²-K/W bis 0,01 m²-K/W hat und die kleidungsseitige Isolierschicht (8B) einen R-Wert hat, der größer als 0,01 m²-K/W ist.

6. Mehrfach verwendbare Kältepackungsvorrichtung nach Anspruch 1, wobei die Gelzusammensetzung (7B) bei einer Temperatur zwischen -5°C und 5°C zu einer festen Form gefriert.

7. Mehrfach verwendbare Kältepackungsvorrichtung nach Anspruch 1, wobei sich das gewellte Material gleichmäßig auf nicht mehr als 10 % Einschnürung dehnt.

8. Mehrfach verwendbare Kältepackungsvorrichtung nach Anspruch 1, wobei sich das gewellte Material gleichmäßig auf nicht mehr als 5 % Einschnürung dehnt.

9. Mehrfach verwendbare Kältepackungsvorrichtung nach Anspruch 1, wobei die Gelpackung (3), wenn sie in Kontakt mit der Haut eines Benutzers gebracht wird, die Hauttemperatur um mindestens 10°C reduziert.

10. Mehrfach verwendbare Kältepackungsvorrichtung nach Anspruch 1, wobei die Gelpackung (3), wenn sie in Kontakt mit der Haut eines Benutzers gebracht wird, eine reduzierte Temperatur der Haut des Benutzers für etwa 15 Minuten bis etwa 60 Minuten aufrechterhält.

11. Mehrfach verwendbare Kältepackungsvorrichtung nach Anspruch 1, wobei die Gelpackung (3), wenn sie in Kontakt mit der Haut eines Benutzers gebracht wird, eine reduzierte Temperatur der Haut des Benutzers für mindestens etwa 15 Minuten aufrechterhält.

12. Mehrfach verwendbare Kältepackungsvorrichtung nach Anspruch 1, wobei die Gelzusammensetzung ferner einen thermochromen Farbstoff umfasst.

13. Mehrfach verwendbare Kältepackungsvorrichtung nach Anspruch 1, wobei jede der einzelnen Zellen der Gelpackung (3) eine Dicke von etwa 2 mm bis 20 mm aufweist.

## Revendications

1. Dispositif de compresse froide multi-usage comprenant :
(i) une enveloppe de fixation comprenant une configuration en Z et ayant une zone de fixation hautement réglable ; et
(ii) une compresse gel multi-cellulaire (3) située entre deux segments de matériau stretch de l'enveloppe de fixation, comprenant
- une couche d'isolation coté corps (6B) ;
- une couche d'isolation côté vêtements (8B) qui s'étend de manière opposée à la couche d'isolation côté corps ; et
- une composition de gel (7B) prévue entre lesdites couches d'isolation côté corps et côté vêtements, dans lequel ladite composition de gel ne comprend pas de composant antigel,
**caractérisé en ce que** les deux segments de matériau stretch comprennent chacun un matériau ondulé qui s'étire de manière uniforme jusqu'à 15% de sa longueur au maximum.

2. Dispositif de compresse froide multi-usage selon la revendication 1, dans lequel l'épaisseur de ladite couche d'isolation côté corps (6B) est inférieure à l'épaisseur de ladite couche d'isolation côté vêtements (8B).

3. Dispositif de compresse froide multi-usage selon la revendication 1 ou 2, dans lequel ladite couche d'isolation côté corps (6B) présente une valeur R de 0,001 m²-K/W à 0,01 m²-K/W.

4. Dispositif de compresse froide multi-usage selon la revendication 1, dans lequel ladite couche d'isolation côté vêtements (8B) présente une valeur R supérieure à 0,01 m²-K/W.

5. Dispositif de compresse froide multi-usage selon la revendication 1, dans lequel ladite couche d'isolation côté corps (6B) présente une valeur R de 0,001 m²-K/W à 0,01 m²-K/W et ladite couche d'isolation côté vêtements (8B) présente une valeur R supérieure à 0,01 m²-K/W.

6. Dispositif de compresse froide multi-usage selon la revendication 1, dans lequel ladite composition de gel (7B) gèle en une forme solide à une température comprise entre -5°C et 5°C.

7. Dispositif de compresse froide multi-usage selon la revendication 1, dans lequel ledit matériau ondulé s'étire de manière uniforme jusqu'à 10% de sa longueur au maximum.

8. Dispositif de compresse froide multi-usage selon la revendication 1, dans lequel ledit matériau ondulé s'étire de manière uniforme jusqu'à 5% de sa longueur au maximum.

9. Dispositif de compresse froide multi-usage selon la revendication 1, dans lequel ladite compresse gel (3), lorsqu'elle est placée en contact avec la peau d'un utilisateur, réduit la température de ladite peau d'au moins 10°C.

10. Dispositif de compresse froide multi-usage selon la revendication 1, dans lequel ladite compresse gel (3), lorsqu'elle est placée en contact avec la peau d'un utilisateur, maintient une température réduite de ladite peau de l'utilisateur pendant environ 15 minutes à environ 60 minutes.

11. Dispositif de compresse froide multi-usage selon la revendication 1, dans lequel ladite compresse gel (3), lorsqu'elle est placée en contact avec la peau d'un utilisateur, maintient une température réduite de ladite peau de l'utilisateur pendant au moins environ 15 minutes.

12. Dispositif de compresse froide multi-usage selon la revendication 1, dans lequel ladite composition de gel comprend en outre un colorant thermochromique.

13. Dispositif de compresse froide multi-usage selon la revendication 1, dans lequel chacune des cellules individuelles de ladite compresse gel (3) présente une épaisseur d'environ 2 mm à 20 mm.
